# EUROPÄISCHE PATENTSCHRIFT

(11) **EP 3 984 365 B1**
(45) Veröffentlichungstag und Bekanntmachung des Hinweises auf die Patenterteilung: **11.09.2024**
(21) Anmeldenummer: 21202775.9
(22) Anmeldetag: 14.10.2021
(51) Int. Cl.: A01N 25/02, A01N 25/34, A01N 37/02, A01N 37/16, A01N 37/36, A01N 59/00, A01P 1/00, A61L 2/18

(54) **MOPPS UND TÜCHER GETRÄNKT MIT EINER WÄSSRIGEN DESINFEKTIONSMITTELZUSAMMENSETZUNG ENTHALTEND PERESSIGSÄURE**
MOPS AND TISSUES SOAKED WITH AN AQUEOUS DISINFECTANT COMPOSITION CONTAINING PERACETIC ACID
SERPILLÈRES ET TISSUS IMBIBÉS D'UNE COMPOSITION DÉSINFECTANTE AQUEUSE CONTENANT DE L'ACIDE PÉRACÉTIQUE

(30) Priorität: 16.10.2020 DE 102020127330
(43) Veröffentlichungstag der Anmeldung: 20.04.2022
(73) Patentinhaber: S.U.MO. GmbH, 21227 Bendestorf (DE)
(72) Erfinder: Knieler, Roland, 21227 Bendestorf (DE)
(74) Vertreter: Müller Schupfner & Partner Patent- und Rechtsanwaltspartnerschaft mbB (Hamburg)

(56) Entgegenhaltungen:
- WO-A1-2016/100818
- WO-A1-2017/165408
- WO-A1-93/02973
- WO-A2-2009/138888
- CA-A1- 2 865 682
- US-A1- 2010 196 503
- US-A1- 2012 213 864
- US-A1- 2018 208 881
- US-B1- 6 387 858
- DONIS GONZÁLEZ I.R. ET AL: "EFFICACY OF POSTHARVEST TREATMENTS FOR REDUCTION OF MOLDS AND DECAY IN FRESH MICHIGAN CHESTNUTS", ACTA HORTICULTURAE, no. 866, 1 June 2010 (2010-06-01), BE, pages 563 - 570, XP055893382, ISSN: 0567-7572, ISBN: 978-90-6605-633-6, Retrieved from the Internet <URL:http://dx.doi.org/10.17660/ActaHortic.2010.866.76> DOI: 10.17660/ActaHortic.2010.866.76
- FRY H S ET AL: "The Action of Hydrogen Peroxide upon Simple Carbon Compounds. III. Glycolic Acid", J. AM. CHEM. SOC., 31 October 1935 (1935-10-31), U.S.A., pages 2260 - 2272, XP093091459, Retrieved from the Internet <URL:https://pubs.acs.org/doi/epdf/10.1021/ja01314a068> [retrieved on 20231013], DOI: 10.1021/ja01314a068

## Beschreibung

Die Erfindung betrifft Tücher und/oder Mopps getränkt mit gebrauchsfertigen wässrigen Desinfektionsmittelzusammensetzungen, die unter anderem Peressigsäure und Wasserstoffperoxid enthalten . Weiterhin betrifft die Erfindung ein nichttherapeutisches Verfahren zur Desinfektion harter Oberflächen mittels Tücher oder Mopps, jeweils getränkt mit der Desinfektionsmittelzusammensetzung und eine entsprechende Verwendung.

### Stand der Technik

Desinfektionsmittelkonzentrate, die vor der Anwendung verdünnt werden müssen, verlieren immer mehr an Bedeutung und sind für viele Anwendungsszenarien nicht gewünscht. Ready-to-Use Anwendungen von Desinfektionsmitteln werden dagegen immer populärer. Die Vorteile sind offensichtlich. Die Prozesssicherheit bei der Anwendung ist erhöht, Ab-, Umfüll- und Verdünnungsprozesse sind nicht mehr notwendig, die Produkte sind sofort einsatzbereit und können auch von ungeschultem Personal und Laien zuverlässig und sicher eingesetzt werden.

Dies bedeutet allerdings, dass die Wirkstoffe in ihrer niedrigeren Anwendungskonzentration im Vergleich zu Konzentraten nicht über einen Anwendungszeitraum von einem Tag, sondern über den gesamten ausgelobten Haltbarkeitszeitraum von z.B. ein bis zwei Jahren bis zum vorgesehenen Verfallsdatum stabil und mit ihrer ausgewiesenen Mindestwirksamkeit annährend gleichwirksam sein müssen. Gebrauchsfertige Anwendungszusammensetzungen müssen also über die ausgelobte Lebensdauer wirksam sein. Dies ist vor allem bei einem Wirkstoff wie Peressigsäure technisch schwer zu lösen, da Verunreinigungen bei Peressigsäure zu Zersetzung (Sauerstoffabspaltung) führen können. Bei einem hochkonzentrierten Konzentrat hat eine geringfügige Zersetzung kaum einen prozentualen Einfluss auf den Gesamtwirkstoffgehalt und die Wirksamkeit des Produktes. Bei niedrig konzentrierten gebrauchsfertigen Lösungen können diese Zersetzungsprozesse schnell zu einer Reduktion der Wirkstoffmenge führen, die sich damit auch nachteilig auf die Wirksamkeit auswirken können oder zu einer unnötigen Überdimensionierung der Anwendungsmenge führt. Häufig wird hier eine Reduzierung der Wirkstoffmenge durch Abbau- bzw. Zerfallsprozesse von über 10% als kritisch angesehen.

Andererseits ist Peressigsäure ein bekannter Bestandteil von Desinfektionsmittelzusammensetzungen mit breiter biozider Wirkung, insbesondere gegenüber Bakterien, Algen, Hefen, Schimmelpilzen, Pilzen, Sporen und Viren.

Aus der WO 93/02973 A1, US 6387858 B1, US 2010/196503 A1 und der Veröffentlichung DONIS GONZALEZ, I.R. et al.: "Efficacy of Postharvest Treatments for Reduction of Molds and Decay in Fresh Michigan Chestnuts" in Proceedings 1st European Congress on Chestnut, Castanea, 2009, Acta Hortic., Bd. 866, 2010, S. 563-569 sind bereits Zusammensetzungen bekannt, welche Peressigsäure, Wasserstoffperoxid, Wasser und Glycolsäure enthalten können, jedoch in anderen Konzentrationen der Bestandteile und nicht in Form von Tüchern oder Mopps getränkt mit jeweils einer solchen Zusammensetzung.

WO 2008/138888 A2 hingegen offenbart vorkonfektionierte Tücher, welche mit einer Desinfektionsmittellösung getränkt sind. Die vorgeschlagenen Lösungen sind jedoch frei von Glycolsäure.

Bisher sind Peressigsäure-Produkte in der Flächendesinfektion in vielen Ländern in Europa noch nicht weit verbreitet, weil in diesen Märkten die Sensibilität für störende Gerüche sehr stark ausgeprägt ist. Peressigsäure-Zusammensetzungen entfalten ohne Modifizierung einen stechenden Geruch.

Aufgabe der vorliegenden Erfindung ist es daher, eine gebrauchsfähige, wässrige Desinfektionsmittelzusammensetzung zur Verfügung zu stellen, die sich zur Flächendesinfektion - insbesondere harter Oberflächen - eignet, geruchsarm ist und eine lange Haltbarkeit aufweist.

### Zusammenfassung der Erfindung

Die Aufgabe wird erfindungsgemäß gelöst mit Tüchern und/oder Mopps gemäß Anspruch 1, ein Verfahren nach Anspruch 13 und eine Verwendung nach Anspruch 14. Bevorzugte Ausführungsformen sind nachfolgend erläutert oder Gegenstand der Unteransprüche.

Die wässrige Desinfektionsmittelzusammensetzung umfasst Peressigsäure, Wasserstoffperoxid und Glycolsäure. Die Desinfektionsmittelzusammensetzung enthält obige Stoffe wie folgt:
a) 0,001 bis 0,2 Gew.%, bevorzugt 0,01 bis 0,1 Gew.%, Peressigsäure;
b) 0,1 bis 7 Gew.%, bevorzugt 0,5 bis 5 Gew.%, insbesondere bevorzugt 1,5 bis 4 Gew.%, Wasserstoffperoxid;
c) 0,001 bis 5 Gew.%, bevorzugt 0,001 bis 3 Gew.%, weiter bevorzugt 0,01 bis 1,5 Gew.% und besonders bevorzugt 0,05 bis 0,75 Gew.%, Glycolsäure; und
d) zumindest 90 Gew.%, vorzugsweise größer 95 Gew.%, Wasser.

Die Angaben beziehen sich jeweils auf die Konzentration des jeweiligen Stoffes in der Zusammensetzung und können beispielsweise durch Titration bestimmt werden.

Eine besonders vorteilhafte Desinfektionsmittelzusammensetzung umfasst:
0.001 bis 0,1 Gew.% Peressigsäure;
0,5 bis 5 Gew.%, insbesondere bevorzugt 1,5 bis 4 Gew.%, Wasserstoffperoxid;
ein Verhältnis (Masse) von Wasserstoffperoxid zu Peressigsäure von mindestens 10 : 1,
0,01 bis 1,5 Gew.% Glycolsäure; und
zumindest 90 Gew.% Wasser.

Die Desinfektionsmittelzusammensetzung besteht vorzugsweise aus den vorgenannten Komponenten zu größer 98 Gew.%, besonders bevorzugt zur größer 99 Gew.%.

Überraschend hat sich gezeigt, dass in verdünnten wässrigen Lösungen bei gleichbleibender Peressigsäure-Konzentration die Stabilität der Mischung mit steigendem Wasserstoffperoxid-Gehalt ansteigt. Ab einem Gewichtsverhältnis von Wasserstoffperoxid zu Peressigsäure von 10:1 liegt die Konzentrationsabnahme von Peressigsäure bei 40°C und nach zwei Monaten je nach Formulierung teilweise deutlich unter dem Grenzwert von 10%.

Der Geruch ist abhängig von der Konzentration der Peressigsäure und wird überraschenderweise von der Konzentration von H₂O₂ beeinflusst, indem die Konzentration des Wasserstoffperoxids gegenüber dem sich einstellenden Verhältnis, das sich schon durch die Zugabe von Peressigsäure ohne Zugabe von Wasserstoffperoxid ergibt, durch Zugabe von weiterem Wasserstoffperoxid erhöht wird. Es ist also - relativ zum Anteil der Peressigsäure - ein hoher Anteil an Wasserstoffperoxid für eine geringe Geruchsintensität hilfreich.

Weiter wird der Geruch reduziert durch den zusätzlichen Einsatz von Glycolsäure, die gleichzeitig eine synergistische Wirkung in Bezug auf die biozide Wirkung in der Zusammensetzung entfaltet.

### Detaillierte Beschreibung der Erfindung

Weiterhin kann die Desinfektionsmittelzusammensetzung enthalten, insbesondere ein oder mehrere nichtionische Tenside. Dies sind z.B. alkoxylierte Fettalkohole, z.B. ethoxyliert (EO), propoxyliert (PO) oder ethoxyliert und propoxyliert (EO, PO).

Nach einer weiteren Ausführungsform handelt es sich bei dem nichtionischen Tensid um alkoxylierte Carbonsäuren oder alkoxylierte Ethercarbonsäuren, z.B. ethoxyliert

Nach einer weiteren Ausführungsform handelt es sich bei dem nichtionischen Tensid um alkoxylierte Carbonsäuren oder alkoxylierte Ethercarbonsäuren, z.B. ethoxyliert (EO), propoxyliert (PO) oder ethoxyliert und propoxyliert (EO, PO). Beispiele für Fettalkohol-Reste sind lineare oder verzweigte C8- bis C18- Alkohole. Der Alkoxlierungsgrad kann 2 bis 16 betragen.

Weiterhin können die Desinfektionsmittelzusammensetzungen Stabilisatoren enthalten. Hierbei handelt es sich beispielsweise um starke Säuren, Sequestriermittel oder Radikalfänger, wie Schwefelsäure, Phosphonsäure, z.B. 1-Hydroxyethylidene(1,1)diphosphonsäure, oder auch Dipicolinsäure oder Butylhydroxytoluole.

Je nach dem vorgesehenen Verwendungszweck kann die Desinfektionsmittelzusammensetzung auch noch Farbstoffe und/oder Parfüms bzw. Geruchsverbesserer oder eine Geruchsmaskierung enthalten. Dies können z.B. Alkohole, Ester und/oder Nitrile sein.

Weiterhin betrifft die Erfindung ein nicht-therapeutisches Verfahren zur Desinfektion harter Oberflächen mit den Tüchern oder Mopps, insbesondere durch Aufbringen der Desinfektionsmittelzusammensetzung auf die harten Oberflächen mittels der Tücher oder Mopp, die mit der Desinfektionsmittelzusammensetzung getränkt sind.

Ein weiterer Gegenstand der Erfindung ist die nicht-therapeutische Verwendung der Zusammensetzung zur Desinfektion von harten Oberflächen mittels der Tücher oder Mopps. Unter harten Oberflächen sind beispielsweise Fußböden, Wände, Tische und Regale, Behälter oder Gerätschaften, wie sie beispielsweise in der Lebensmittel- und Agrarlebensmittelindustrie oder bei der Zubereitung, Verarbeitung und Verpackung von Lebensmitteln und Getränken, in der Bioindustrie, der pharmazeutischen Industrie, der Kosmetikindustrie, in Reinräumen, in Gewächshäusern und Pflanzen- oder Tierzuchtgebäuden oder auch auf dem Gebiet der Hygiene und Gesundheit verwendet werden. Ein besonderes Anwendungsgebiet für die Desinfektionsmittelzusammensetzung ist die Desinfektion von Krankenhausräumen, Arztpraxen und Pflegeheimen.

Die zu behandelnden Oberflächen können unterschiedlich sein, insbesondere harte Oberflächen; genannt seien beispielsweise gewerbliche oder öffentliche Einrichtungen, Fußböden, Gerätschaften, Werkzeuge und Utensilien, Behältnisse wie Tanks, konservierende Verpackungen, Rohre und Schläuche.

Die zu behandelnde Oberfläche kann aus einem beliebigen Material und insbesondere aus Glas, Aluminium oder rostfreiem Stahl, Keramik oder organischem Polymer, wie beispielsweise Polyethylen, Polypropylen, Polycarbonat, Polyamid, Polyester, wie Polyethylenterephthalat (PET), Polyurethan, Fluorpolymeren, wie PTFE, PVDF und PFA, und Polyvinylchlorid (PVC) bestehen.

Nach einer Ausgestaltung der vorliegenden Erfindung wird die wässrige Desinfektionsmittelzusammensetzung von einem porösen Material, wie einem Schwamm, Papier, Bürstenfasern oder Stoff, absorbiert, wobei man eine gebrauchsfertige Formulierung auf einem festen Träger, wie beispielsweise einem Tuch, Vliesstoff, Bürsten oder einem anderen textilen Material, erhält. Die Tücher können z.B. aus Cellulose, Cellulosederivate, Viskose, PET, PE, und/oder PP sein. Insbesondere geeignet sind Mopps, z.B. in Form austauschbarer Reinigungsbezüge, z.B. mit Fransen oder Schlingen. Der Mopp kann z.B. auf oder an einer an einem Stiel, ggf. drehbar, befestigten Grundplatte befestigt bzw. mit einem Klettverschluss angeheftet sein. Die Mopps sind vorzugsweise mehrlagig, wobei die Lagen aus unterschiedlichen Materialien bestehen können.

Die Mopps und Tücher werden vorkonfektioniert, d.h. mit der Desinfektionsmittelzusammensetzung getränkt und sind z.B. in Kunststoffbeuteln oder -gefäßen angeboten. Diese können auch für den Einmalgebrauch vorgesehen sein.

Überraschenderweise hat der Gehalt an Wasserstoffperoxid und damit das Verhältnis von Wasserstoffperoxid zu Peressigsäure (PAA) bei gleichbleibender Peressigsäure-Konzentration Einfluss auf den Geruch. Der Geruch wurde mit Probanden in Anlehnung an VDI 3882, Blatt 2, Olfaktometrie: Bestimmung der Geruchsintensität mit einer Kategorieskala von 0 bis 5, in 0,25er Abstufung, 0=geruchlos, 5=sehr starker Geruch bestimmt, angegeben wird der arithmetische Mittelwert.

**Tabelle 1 Geruchtests (%-Angaben in Gewichtsprozent bzw. Gewichtsverhältnis)**

| PAA | A1 | A2 | A3 |
|---|---|---|---|
| PAA | 0,20% | 0,20% | 0,20% |
| Wasserstoffperoxid | 1% | 2% | 3% |
| Verhältnis Wasserstoffperoxid zu PAA | 5 | 10 | 15 |
| Geruchsintensität | 4,38 | 3,88 | 3,50 |

| | B1 | B2 | B3 |
|---|---|---|---|
| PAA | 0,10% | 0,10% | 0,10% |
| Wasserstoffperoxid | 0,7% | 1,5% | 3% |
| Verhältnis Wasserstoffperoxid zu PAA | 7 | 10 | 30 |
| Geruchsintensität | 3,58 | 3,25 | 2,46 |

| | C1 | C2 | C3 |
|---|---|---|---|
| PAA | 0,05% | 0,05% | 0,05% |
| Wasserstoffperoxid | 0,5% | 2% | 3% |
| Verhältnis Wasserstoffperoxid zu PAA | 10 | 40 | 60 |
| Geruchsintensität | 2,46 | 2,25 | 1,75 |

Der Geruch ist abhängig von der Konzentration der PAA und überraschenderweise auch von der Konzentration an Wasserstoffperoxid. Es wurde gefunden, dass ein geringer Anteil an Peressigsäure (PAA) und ein hoher Anteil an Wasserstoffperoxid die Geruchsintensität minimiert. Der Bestandteil Glycolsäure verändert den Geruch nicht und wurde daher bei dem Geruchstest nicht berücksichtigt.

Überraschenderweise erhöht Glycolsäure die Wirksamkeit der Formulierung überproportional, so dass die erfindungsgemäße Zusammensetzung erlaubt, den Peressigsäure-Gehalt niedrig zu halten bzw. zu minimieren, womit der Geruch weiter reduziert werden kann. Dies ist der synergistischen Wirkung der Kombination der eingesetzten Stoffe Peressigsäure, Wasserstoffperoxid und Glycolsäure zuzuschreiben.

Die mikrobiologischen Ergebnisse sind Screening-Ergebnisse in Anlehnung an EN 17126 (C. diff. Sporen) und EN 14476 (Noroviren, Adenoviren), dargestellt in log10-Reduktion.

Die Herstellung der Mischung für die mikrobiologische Prüfung erfolgte wie folgt:

Wasser wurde vorgelegt, Wasserstoffperoxid (Persynth 300 LC, 30 Gew.-% Wasserstoffperoxid) wurde unter Rühren zugeben, Peressigsäure (Peraclean 5 mit 4,8 Gew.-% Peressigsäure und 26 Gew.-% Wasserstoffperoxid) wurde in einem Mengenverhältnis zugeben, so dass die unten angegebenen Endkonzentrationen erhalten wurden. Sodann wurde unter weiterem Rühren 0,1% Glycolsäure zugeben und danach 0,2% einer ethoxylierten Ethercarbonsäure und 0,1 Gew.-% 1-Hydroxyethylidene(1,1)diphosphonsäure. Das Gemisch hatte einen pH-Wert von 2,1 - 2,5 bei Raumtemperatur (21 °C).

**Tabelle 2**

| Rezeptur Nr. | 1 | 2 | 3 | 4 | 5 |
|---|---|---|---|---|---|
| Gehalt Peressigsäure [Gew.%] | 0,1 | 0,05 | 0,1 | 0,05 | 0 |
| Gehalt Glycolsäure [Gew.%] | 0 | 0 | 0,1 | 0,1 | 0,1 |
| Gehalt H₂O₂ [Gew.%] | 3 | 3 | 3 | 3 | 0 |
| Noroviren | 3,25 | 2,88 | >4 | >4 | 0,38 |
| Adenoviren | 2,75 | 2,13 | >4 | 3,88 | 0 |

Die synergistische Wirksamkeit bei der Virusinhibierung ist zu erkennen.

**Tabelle 3**

| Rezeptur Nr. | 3 | 4 |
|---|---|---|
| Gehalt Peressigsäure [Gew.%] | 0,1 | 0,05 |
| Gehalt Glycolsäure [Gew.%] | 0,1 | 0,1 |
| Gehalt H₂O₂ [%] | 3 | 3 |
| C. diff. | >5 | >5 |

Gleiches gilt für die Sporenwirksamkeit gegen C. diff.-Sporen (Clostridioides Difficile). Der Gehalt an Peressigsäure und Wasserstoffperoxid wurde durch Titration bestimmt. Die Gehaltsbestimmung von Wasserstoffperoxid und Peressigsäure erfolgte mittels einer 2-Stufentitration, die die unterschiedliche Reaktionsgeschwindigkeit von Wasserstoffperoxid und Peressigsäure nutzt. In der ersten Stufe wurde das Wasserstoffperoxid permanganometrisch bestimmt und in der zweiten Stufe wurde die Peressigsäure iodometrisch titriert.

## Patentansprüche

1. Tücher und/oder Mopps jeweils getränkt mit einer Desinfektionsmittelzusammensetzung und gebrauchsfertig vorkonfektioniert und verpackt, die Desinfektionsmittelzusammensetzung umfassend
0,001 bis 0,2 Gew.% Peressigsäure,
0,1 bis 7 Gew.% Wasserstoffperoxid,
zumindest 90 Gew.% Wasser und
0,001 bis 5 Gew.% Glycolsäure.

2. Tücher und/oder Mopps nach Anspruch 1, wobei die Desinfektionsmittelzusammensetzung
0,001 bis 0,1 Gew.% Peressigsäure umfasst.

3. Tücher und/oder Mopps nach Anspruch 1 oder 2 , wobei die Desinfektionsmittelzusammensetzung
0,5 bis 5 Gew.%, bevorzugt 1,5 bis 4 Gew.% Wasserstoffperoxid umfasst.

4. Tücher und/oder Mopps nach zumindest einem der vorhergehenden Ansprüche, wobei die Desinfektionsmittelzusammensetzung
0,01 bis 1,5 Gew.% Glycolsäure und bevorzugt 0,05 bis 0,75 Gew.% Glycolsäure umfasst.

5. Tücher und/oder Mopps nach zumindest einem der vorhergehenden Ansprüche, wobei das Verhältnis (Masse) von Wasserstoffperoxid zu Peressigsäure mindestens 10 : 1 beträgt, bevorzugt 10 : 1 bis 500 : 1, besonders bevorzugt 10:1 bis 100 : 1 und ganz besonders bevorzugt 15 : 1 bis 80 : 1.

6. Tücher und/oder Mopps nach zumindest einem der vorhergehenden Ansprüche, wobei die Desinfektionsmittelzusammensetzung zumindest 95 Gew.%, Wasser umfasst.

7. Tücher und/oder Mopps nach zumindest einem der vorhergehenden Ansprüche , wobei die Desinfektionsmittelzusammensetzung
0,001 bis 0,1 Gew.% Peressigsäure;
0,5 bis 5 Gew.%, bevorzugt 1,5 bis 4 Gew.%, Wasserstoffperoxid;
ein Verhältnis (Masse) von Wasserstoffperoxid zu Peressigsäure von mindestens 10 : 1;
0,01 bis 1,5 Gew.% Glycolsäure; und
zumindest 90 Gew.% Wasser umfasst.

8. Tücher und/oder Mopps nach zumindest einem der vorhergehenden Ansprüche, wobei die Desinfektionsmittelzusammensetzung weiterhin ein oder mehrere Additive enthält ausgewählt aus der Gruppe enthaltend Tenside, insbesondere nicht-ionische Tenside, C2- bis C4- Alkohole, Säuerungsmittel auf Phosphorbasis, Komplexbildner, Korrosionsinhibitoren und geruchsoptimierende Stoffe, insbesondere eine Phosphonsäure.

9. Tücher und/oder Mopps nach zumindest einem der vorhergehenden Ansprüche, wobei die Desinfektionsmittelzusammensetzung aus den Komponenten der Ansprüche 1 bis 8 zu größer 98 Gew.%, vorzugsweise zur größer 99 Gew.% besteht.

10. Tücher und/oder Mopps nach zumindest einem der vorhergehenden Ansprüche, wobei die Desinfektionsmittelzusammensetzung einen pH von 2,1 bis 2,5 bei 25°C aufweist.

11. Tücher und/oder Mopps nach zumindest einem der vorhergehenden Ansprüche umfassend oder bestehend aus Cellulose, Cellulosederivate, Viskose, Polyethylenterephthalat, Polyethylen und/oder Polypropylen.

12. Mopps nach zumindest einem der vorhergehenden Ansprüche, wobei die Mopps austauschbare Reinigungsbezüge sind und der Mopp bzw. Reinigungsbezug auf oder an einer an einem Stiel, ggf. drehbar, befestigten Grundplatte befestigt bzw. mit einem Klettverschluss angeheftet ist.

13. Nicht-therapeutische Verfahren zur Desinfektion harter Oberflächen mit den Tüchern oder Mopps nach zumindest einem der vorhergehenden Ansprüche durch Aufbringen der Desinfektionsmittelzusammensetzung auf die harten Oberflächen mittels der Tücher oder Mopps, die mit der Desinfektionsmittelzusammensetzung getränkt sind.

14. Nicht-therapeutische Verwendung der Tücher und/oder Mopps nach zumindest einem der Ansprüche 1 bis 12 zur Desinfektion harter Oberflächen.

## Claims

1. Tissues and/or mops each impregnated with a disinfectant composition and ready to use pre-confectioned and packaged, the disinfectant composition comprising
0.001 to 0.2 wt. % peracetic acid,
0.1 to 7 wt.% hydrogen peroxide,
at least 90 wt. % water and
0.001 to 5 wt.% glycolic acid.

2. Tissues and/or mops according to claim 1, wherein the disinfectant composition comprises
0.001 to 0.1 wt. % peracetic acid.

3. Tissues and/or mops according to claim 1 or 2, wherein the disinfectant composition comprises
0.5 to 5 wt.%, preferably 1.5 to 4 wt.% hydrogen peroxide.

4. Tissues and/or mops according to at least one of the preceding claims, wherein the disinfectant composition comprises
0.01 to 1.5 wt.% glycolic acid and preferably 0.05 to 0.75 wt.% glycolic acid.

5. Tissues and/or mops according to at least one of the preceding claims, wherein the ratio (mass) of hydrogen peroxide to peracetic acid is
at least 10 : 1, preferably 10 : 1 to 500 : 1, more preferably 10 : 1 to 100 : 1 and most preferably 15 : 1 to 80 : 1.

6. Tissues and/or mops according to at least one of the preceding claims, wherein the disinfectant composition comprises
at least 95 wt.% water.

7. Tissues and/or mops according to at least one of the preceding claims, wherein the disinfectant composition comprises
0.001 to 0.1 wt.% peracetic acid;
0.5 to 5 wt.%, preferably 1.5 to 4 wt.%, hydrogen peroxide;
a ratio (mass) of hydrogen peroxide to peracetic acid of at least 10 : 1;
0.01 to 1.5 wt.% glycolic acid; and
at least 90 wt.% water.

8. Tissues and/or mops according to at least one of the preceding claims, wherein the disinfectant composition further comprises one or more additives selected from the group comprising surfactants, in particular non-ionic surfactants, C2- to C4- alcohols, phosphorus-based acidifying agents, complexing agents, corrosion inhibitors and odour-optimising substances, in particular a phosphonic acid.

9. Tissues and/or mops according to at least one of the preceding claims, wherein the disinfectant composition consists of the components of claims 1 to 8 to more than 98 wt.%, preferably to more than 99 wt.%.

10. Tissues and/or mops according to at least one of the preceding claims, wherein the disinfectant composition has a pH of from 2.1 to 2.5 at 25°C.

11. Tissues and/or mops according to at least one of the preceding claims comprising or consisting of cellulose, cellulose derivatives, viscose, polyethylene terephthalate, polyethylene and/or polypropylene.

12. Mops according to at least one of the preceding claims, wherein the mops are exchangeable cleaning covers and the mop and/or cleaning cover is attached to or on a base plate attached to a handle, optionally rotatably, and/or is attached with a hook- and-loop fastener.

13. A non-therapeutic method of disinfecting hard surfaces with the tissues or mops according to at least one of the preceding claims by applying the disinfectant composition to the hard surfaces by means of the tissues or the mops impregnated with the disinfectant composition.

14. Non-therapeutic use of the tissues and/or mops according to at least one of claims 1 to 12 for disinfecting hard surfaces.

## Revendications

1. Tissus et/ou serpillières imbibés respectivement d'une composition désinfectante, préparés à l'avance et conditionnés prêts à l'emploi, la composition désinfectante comprenant
0,001 à 0,2 % en poids d'acide peracétique,
0,1 à 7 % en poids de peroxyde d'hydrogène,
au moins 90 % en poids d'eau et
0,001 à 5 % en poids d'acide glycolique.

2. Tissus et/ou serpillières selon la revendication 1, dans lesquels la composition désinfectante comprend
0,001 à 0,1 % en poids d'acide peracétique.

3. Tissus et/ou serpillières selon la revendication 1 ou 2, dans lesquels la composition désinfectante comprend
0,5 à 5 % en poids, de préférence 1,5 à 4 % en poids de peroxyde d'hydrogène.

4. Tissus et/ou serpillières selon au moins l'une des revendications précédentes, dans lesquels la composition désinfectante comprend
0,01 à 1,5 % en poids d'acide glycolique et de préférence 0,05 à 0,75 % en poids d'acide glycolique.

5. Tissus et/ou serpillières selon au moins l'une des revendications précédentes, dans lesquels le rapport (masse) du peroxyde d'hydrogène sur l'acide peracétique est d'au moins 10 : 1, de préférence 10: 1 à 500 : 1, de manière particulièrement préférée de 10 : 1 à 100 : 1 et de manière toute particulièrement préférée de 15 : 1 à 80 : 1.

6. Tissus et/ou serpillières selon au moins l'une des revendications précédentes, dans lesquels la composition désinfectante comprend au moins 95 % en poids d'eau.

7. Tissus et/ou serpillières selon au moins l'une des revendications précédentes, dans lesquels la composition désinfectante comprend
0,001 à 0,1 % en poids d'acide peracétique ;
0,5 à 5 % en poids, de préférence 1,5 à 4 % en poids de peroxyde d'hydrogène ;
un rapport (masse) du peroxyde d'hydrogène sur l'acide peracétique d'au moins 10 : 1 ;
0,01 à 1,5 % en poids d'acide glycolique ; et
au moins 90 % en poids d'eau.

8. Tissus et/ou serpillières selon au moins l'une des revendications précédentes, dans lesquels la composition désinfectante contient en outre un ou plusieurs additifs choisis dans le groupe contenant les tensioactifs, en particulier les tensioactifs non ioniques, les alcools en C2 à C4, les agents acidifiants à base de phosphore, les agents complexants, les inhibiteurs de corrosion et les substances optimisant l'odeur, en particulier un acide phosphorique.

9. Tissus et/ou serpillières selon au moins l'une des revendications précédentes, dans lesquels la composition désinfectante se compose des composants selon les revendications 1 à 8 à plus de 98 % en poids, de préférence à plus de 99 % en poids.

10. Tissus et/ou serpillières selon au moins l'une des revendications précédentes, dans lesquels la composition désinfectante présente un pH de 2,1 à 2,5 à 25°C.

11. Tissus et/ou serpillières selon au moins l'une des revendications précédentes, comprenant ou se composant de cellulose, de dérivés de cellulose, de viscose, de polyéthylène téréphtalate, de polyéthylène et/ou de polypropylène.

12. Serpillières selon au moins l'une des revendications précédentes, les serpillières étant des housses de nettoyage remplaçables et la serpillière et/ou la housse de nettoyage étant fixée sur ou à une plaque de base fixée à un manche, le cas échéant rotative, et/ou attachée avec une fermeture de type Velcro.

13. Procédé non thérapeutique de désinfection de surfaces dures avec les tissus ou serpillières selon au moins l'une des revendications précédentes par application de la composition désinfectante sur les surfaces dures au moyen des tissus ou des serpillières qui sont imbibés de la composition désinfectante.

14. Utilisation non thérapeutique des tissus et/ou serpillières selon au moins l'une des revendications précédentes 1 à 12 pour la désinfection de surfaces dures.
